(19) **Europäisches Patentamt / European Patent Office / Office européen des brevets**

(11) **EP 4 710 942 A1**

(12) **EUROPEAN PATENT APPLICATION**
published in accordance with Art. 153(4) EPC

(43) Date of publication:
18.03.2026 Bulletin 2026/12

(21) Application number: 24825330.4

(22) Date of filing: 21.06.2024

(51) International Patent Classification (IPC):
*A61K 47/46* (2006.01)  *A61K 47/62* (2017.01)
*A61K 47/69* (2017.01)  *A61K 31/136* (2006.01)
*A61K 31/713* (2006.01)  *A61K 9/52* (2006.01)
*A61P 35/00* (2006.01)

(86) International application number:
PCT/CN2024/100604

(87) International publication number:
WO 2024/260443 (26.12.2024 Gazette 2024/52)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 21.06.2023 CN 202310738539

(71) Applicant: Jenkem Technology Co. Ltd. (Tianjin)
Tianjin 300462 (CN)

(72) Inventors:
- HE, Huining
  Tianjin 300462 (CN)
- YANG, Mengting
  Tianjin 300462 (CN)
- ZHU, Mengxi
  Tianjin 300462 (CN)
- YU, Fei
  Tianjin 300462 (CN)
- SUN, Lu
  Tianjin 300462 (CN)
- WANG, Jianxin
  Tianjin 300462 (CN)

(74) Representative: HGF
HGF Limited
4th Floor, 1 City Square
Leeds LS1 2ES (GB)

Remarks:
The complete document including Reference Table(s) and the Sequence Listing(s) can be downloaded from the EPO website

(54) **PREPARATION FOR SIRNA AND DRUG CO-DELIVERY SYSTEM FOR EFFICIENTLY TARGETING GLIOMA INITIATING CELLS, AND USE**

(57) Preparation for an siRNA and drug co-delivery system for efficiently targeting glioma initiating cells, and a use; DVAP targeting peptide modification is performed on exosomes by utilizing a click chemistry method, siRNA and mitoxantrone are loaded into the exosomes, and construction is performed to obtain a target-modified drug carrier exosome DVAP-Exo/siRNA/MIT. The target-modified exosome co-carries chemotherapeutic mitoxantrone, which has a strong killing effect on glioma initiating cells and can trigger immunogenic cell death, and a small interfering nucleic acid (siNotch 1), which is capable of reducing glioma initiating cell stemness; targeted tracking and killing of residual glioma initiating cells and tumor cells are achieved, and a new avenue is provided for glioblastoma treatment.

FIG. 1

## Description

### TECHNICAL FIELD

The present invention pertains to the field of biopharmaceuticals, and particularly relates to preparation and use of an siRNA and drug co-delivery system for efficiently targeting glioma initiating cells.

### BACKGROUND

Glioblastoma (GBM) is a highly malignant and highly invasive primary tumor. It is difficult to achieve 100% resection of tumor cells during surgery, and the residual glioma initiating cells (GICs) and glioma cells are the root causes of tumor recurrence. Therefore, finding methods that can simultaneously target and kill residual GICs and glioma cells is key to inhibiting brain glioma recurrence. The standard regimen for clinical treatment of GBM is surgical resection, supplemented by postoperative radiotherapy and chemotherapy. Currently, there are some relatively established treatment options for brain glioma, including temozolomide (TMZ), procarbazine, lomustine, and vincristine (PCV), and mammalian target of rapamycin (mTOR) inhibitors. Temozolomide is a first-line chemotherapeutic drug for treating glioma. It is a small molecule alkylating agent. In 2005, a study showed that TMZ combined with radiotherapy significantly prolonged the median survival of glioblastoma patients compared with radiotherapy alone. However, due to the emergence of drug resistance, the median survival rate of glioma patients remains very low. PCV is a combination of chemotherapeutic drugs. Due to its potential to alleviate clinical symptoms, it has a good therapeutic effect in the treatment of recurrent low-grade gliomas. mTOR inhibitors inhibit the activity of activated mTOR and its downstream proteins by directly acting on the mTOR target, thereby interfering with the growth, differentiation, and metabolism of tumor cells. Temsirolimus and everolimus are small molecule inhibitors that can block mammalian mTOR, thus regulating the growth and progression of glioma. However, due to the development of resistance mechanisms, clinical trials of mTOR inhibitors have shown very limited efficacy against glioma. In addition to the established treatment strategies described above, some promising treatment strategies have been developed, including emerging strategies such as tumor treating fields, immunotherapy, and tyrosine kinase inhibitors, but none have yet achieved good therapeutic effects in clinical trials. Mitoxantrone (MIT) has an extremely strong killing effect on GICs and brain glioma cells, inducing cell death by interfering with nucleic acid synthesis and double-strand break repair. Furthermore, the Notch 1 pathway is highly activated in GICs and plays an important role in regulating behaviors, such as proliferation and stemness maintenance, of GICs. Anticancer drugs such as doxorubicin and paclitaxel have many limitations, including poor solubility in aqueous solutions, systemic non-specific distribution, and cancer cell drug resistance. Therefore, cancer treatment strategies based on single drugs are inadequate.

### SUMMARY

In order to solve the technical problems described above, the present invention provides preparation and use of an siRNA and drug co-delivery system for efficiently targeting glioma initiating cells.

The technical solution adopted in the present invention is an exosome drug delivery system loaded with mitoxantrone and siRNA, and the $^D$VAP targeting peptide-modified exosome is loaded with siRNA and mitoxantrone (MIT).

Preferably, sequences of siRNA are set forth in SEQ ID No. 1 and SEQ ID No. 2. Provided is a construction method for the exosome drug delivery system loaded with mitoxantrone and siRNA, which comprises the following steps:

step 1: modifying an exosome with a $^D$VAP targeting peptide to obtain $^D$VAP-Exo;
step 2: loading siRNA and MIT into $^D$VAP-Exo to obtain $^D$VAP-Exo/MIT/siRNA.

Preferably, in step 1, DBCO is firstly linked to the outer surface of the exosome to obtain DBCO-Exo, and then $^D$VAP-N$_3$ and DBCO-Exo are mixed and incubated to obtain $^D$VAP targeting peptide-modified exosome $^D$VAP-Exo.

Preferably, 10 $\mu$M DBCO-sulfo-NHS is dissolved in DMSO, the exosome resuspended in PBS is added, the mixture is reacted at 25 °C and 120 rpm for 4 h, and excess DBCO-sulfo-NHS is removed to obtain DBCO-Exo;

$^D$VAP-N$_3$ modified with an azido group is incubated with DBCO-Exo at 4 °C on a shaker at 100 rpm to eliminate free peptides, and $^D$VAP-Exo is obtained.

Preferably, in step 2, siRNA and MIT are loaded into the modified exosome by adopting the co-incubation combined with transfection reagent method.

Preferably, the method specifically comprises:

step 1: uniformly mixing MIT, $^D$VAP-Exo, and siRNA according to a ratio of $^D$VAP-Exo:MIT:siRNA = 10:8:1, adding an Exo-fect transfection reagent, incubating the mixture at 37 °C for 20 min, then adding an ExoQuick-TC reagent, and letting the mixture stand on ice for 30 min;
step 2: centrifuging the mixture at 4 °C and 13000 rpm for 3 min, discarding the supernatant, retaining the precipitate, adding an appropriate amount of PBS for resuspension, and dialyzing the obtained system in a dialysis bag for 10 h to obtain $^D$VAP-Exo/MIT/siRNA.

Provided is use of the exosome drug delivery system loaded with mitoxantrone and siRNA in preparing a medicament against glioma.

Preferably, the exosome drug delivery system loaded with mitoxantrone and siRNA is capable of acting on residual glioma initiating cells and/or glioma cells.

The present invention has the advantages and positive effects as follows: a drug delivery system formed by using exosomes with a targeted modification to co-load mitoxantrone, which is a chemotherapeutic drug that can trigger immunogenic cell death, and siRNA, which is a small interfering nucleic acid (siNotch 1) that can reduce the stemness of glioma initiating cells, has a strong killing effect on glioma initiating cells, so that the targeted tracking and killing of residual glioma initiating cells and tumor cells are achieved, and a new strategy for the treatment of glioblastoma is provided.

## BRIEF DESCRIPTION OF THE DRAWING

FIG. 1: The exosome-targeted delivery system.

FIG. 2: The characterization of exosomes; (A): the transmission electron microscopy image of exosomes; (B): the particle size distribution graph of exosomes; (C): the Western blot images of surface-specific molecules of cells and exosomes.

FIG. 3: The characterization of the exosome-targeted drug delivery system; (A): the flow cytometry analysis graph of the targeted modification of exosomes; (B): the transmission electron microscopy images of the drug delivery system; (C): the particle size distribution graphs of the drug delivery system; scale bar: 100 nm.

FIG. 4: The results of siRNA and MIT stability investigation; (A): the agarose gel electrophoresis images after incubation of siRNA and RNase A for different time periods; (B): the release curve of MIT under the condition of pH = 7.4.

FIG. 5: The results of phenotypic identification of glioma initiating cell spheres; (A): the Western blot images of U87 cell sphere-specific molecules; (B): the immunofluorescence images of CD133 and Notch 1 of U87 cell spheres; scale bar: 200 μm.

FIG. 6: The results of *in vitro* targeting evaluation; (A): the uptake images of each administration group in HA1800 cells; (B): the uptake images of each administration group in U87 cells; exosomes labeled with DiI (red), cell nuclei labeled with DAPI (blue); scale bar: 20 μm.

FIG. 7: The uptake images of each administration group in U87 cell spheres; exosomes labeled with DiI (red); scale bar: 100 μm.

FIG. 8: The uptake of the drug-loaded system in U87 cells; (A): the uptake of each administration group in U87 cells; (B): the results of quantitative analysis of fluorescence of Cy3-siRNA and MIT in U87 cells; scale bar: 20 μm; cell nuclei labeled with DAPI (blue), siRNA labeled with Cy3 (red), fluorescence of MIT itself (green); **$P < 0.01$, ***$P < 0.001$, n = 3.

FIG. 9: The uptake of the drug-loaded system in U87 cells; (A): the uptake of each administration group in U87 cell spheres; (B): the results of quantitative analysis of fluorescence of Cy3-siRNA and MIT in U87 cell spheres; scale bar: 200 μm; cell nuclei labeled with DAPI (blue), siRNA labeled with Cy3 (red), fluorescence of MIT itself (green); **$P < 0.01$, n = 3.

FIG. 10: The uptake mechanism of exosomes with a targeted modification in U87 cells; (A): the uptake mechanism of VE in U87 cells; (B): the results of quantitative analysis of uptake mechanisms; exosomes labeled with DiI (red), cell nuclei labeled with DAPI (blue); scale bar: 20 μm; ***$P < 0.001$, n = 3.

FIG. 11: The uptake mechanism of exosomes with a targeted modification in U87 cell spheres; (A): the uptake mechanism of VE in U87 cell spheres; (B): the results of quantitative analysis of uptake mechanisms; exosomes labeled with DiI (red), cell nuclei labeled with DAPI (blue); *$P < 0.05$, n = 3.

FIG. 12: The analysis results of co-localization of siRNA, MIT, and lysosomes in U87 cells; (A): the confocal microscopy images of U87 cells after uptake of VEMR; (B): the quantitative analysis results of co-localization coefficient by ImageJ; scale bar: 20 μm; lysosomes labeled with Lysotracker (green), exosomes labeled with DiI (red), cell nuclei labeled with DAPI (blue), fluorescence of MIT itself (purple); *$P < 0.05$, **$P < 0.01$, n = 3.

FIG. 13: The analysis results of co-localization of siRNA, MIT, and lysosomes in U87 cell spheres; (A): the confocal microscopy images of U87 cell spheres after uptake of VEMR; (B): the quantitative analysis results of co-localization coefficient by ImageJ; scale bar: 200 $\mu$m; lysosomes labeled with Lysotracker (green), exosomes labeled with Dil (red), cell nuclei labeled with DAPI (blue), fluorescence of MIT itself (purple); *$P < 0.05$, **$P < 0.01$, n = 3.

FIG. 14: The evaluation results of the gene silencing effect of $^D$VAP-Exo/siRNA in U87 cell spheres and GL261 cells; (A): the mRNA expression level of Notch 1 detected by qRT-PCR; (B): the protein expression level of Notch 1 detected by Western blot; *$P < 0.05$, **$P < 0.01$, ***$P < 0.001$, n = 3.

FIG. 15: The results of the safety of the exosome-targeted drug delivery system on HUVEC and U87 cells detected by the CCK-8 method.

FIG. 16: The results of evaluation of *in vitro* cell proliferation; (A): the effect of the exosome-targeted drug delivery system on U87 cell proliferation detected by the CCK-8 method; (B): the effect of the exosome-targeted drug delivery system on U87 cell sphere proliferation detected by the CCK-8 method; ns: no significant difference, *$P < 0.05$, **$P < 0.01$, ***$P < 0.001$, n = 3.

FIG. 17: The results of evaluation of cell migration ability; (A): the scratch images and the results of distance quantitative analysis of each administration group in U87 cells; (B): the scratch images and the results of distance quantitative analysis of each administration group in U87 cell spheres; scale bar: 200 $\mu$m; ns: no significant difference, *$P < 0.05$, ***$P < 0.001$, n = 3.

FIG. 18: The flow cytometry analysis graphs showing the effects of each administration group on U87 cell apoptosis.

FIG. 19: The flow cytometry analysis graphs showing the effects of each administration group on U87 cell sphere apoptosis.

FIG. 20: The results of the small animal *in vivo* imaging experiment; (A): the scanning images of small animal *in vivo* imaging; (B): the results of quantitative analysis of fluorescence signals; **$P < 0.01$, n = 5.

FIG. 21: The results of H&E staining of brain tissue sections; (A): images showing H&E staining of brain tissue sections of mice in each group; (B): the results of quantitative analysis of tumor size in each group; *$P < 0.05$, **$P < 0.01$, ***$P < 0.001$, n = 5.

FIG. 22: Images showing TUNEL staining of brain tissue sections; (A): TUNEL staining of mouse brain tissue sections; (B): the results of quantitative fluorescence analysis; cell nuclei (blue), apoptotic cell nucleus fragments (green); scale bar: 20 $\mu$m; *$P < 0.05$, **$P < 0.01$, ***$P < 0.001$, n = 5.

FIG. 23: The results of changes in the body weight of mice in different administration groups.

FIG. 24: Images showing H&E staining of mouse major organ sections, scale bar: 100 $\mu$m.

FIG. 25: The results of detection of mouse liver and kidney function indicators.

## DETAILED DESCRIPTION

Hereinafter, the embodiments of the present invention will be described with reference to the accompanying drawings.

The present invention relates to preparation and use of an siRNA and drug co-delivery system for efficiently targeting glioma initiating cells. Exosomes modified with a $^D$VAP targeting peptide and loaded with siRNA and MIT form an exosome drug delivery system loaded with mitoxantrone and siRNA. The drug delivery system utilizes exosomes with a targeted modification to co-load the chemotherapeutic drug mitoxantrone, which has a strong killing effect on glioma initiating cells and can trigger immunogenic cell death, and siNotch 1, which can reduce the stemness of glioma initiating cells (GICs), so that the targeted tracking and killing of residual glioma initiating cells and tumor cells are achieved, and siNotch 1 and MIT are delivered safely and efficiently, thereby inhibiting the recurrence of brain glioma and achieving the purpose of a combined gene-and-chemotherapy treatment for brain glioma.

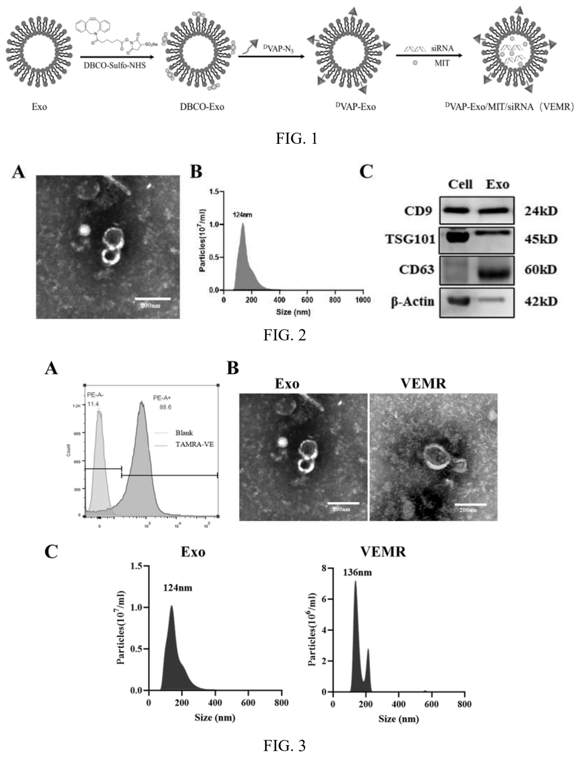

The construction method for the exosome drug delivery system loaded with mitoxantrone and siRNA: Firstly, an exosome is separated by the differential ultracentrifugation method, a $^D$VAP targeting peptide is then covalently conjugated to the surface of the exosome by the click chemistry method to form $^D$VAP-Exo, and siRNA and mitoxantrone are loaded into $^D$VAP-Exo by the co-incubation combined with transfection reagent method to obtain the drug-loaded exosome with a targeted modification ($^D$VAP-Exo/siRNA/MIT, VEMR). The preparation process of the exosome-targeted drug delivery system is shown in FIG. 1.

Specifically, the process comprises the following steps:

step 1: modifying an exosome with a $^D$VAP targeting peptide to obtain $^D$VAP-Exo, wherein DBCO is firstly linked to the outer surface of the exosome to obtain DBCO-Exo, then $^D$VAP-N$_3$ and DBCO-Exo are mixed and incubated, followed by incubation at 4 °C on a shaker at 100 rpm to eliminate free peptides, and the $^D$VAP targeting peptide-modified exosome $^D$VAP-Exo is obtained.

Step 2: loading siRNA and MIT into the exosome, wherein siRNA and MIT are loaded into the modified exosome by adopting the co-incubation combined with transfection reagent method, and $^D$VAP-Exo/MIT/siRNA is obtained after purification.

Tests on the obtained drug-loaded exosome with a targeted modification confirm that the drug-loaded exosome maintains intact structure and unimpaired morphology, with a slight increase in particle size. The exosome effectively protects siRNA from degradation by nucleases. Furthermore, MIT in the drug-loaded system is released steadily within 48 h, demonstrating good release stability. A $^D$VAP peptide specifically binds to the GRP78 protein receptor. The uptake of exosomes with a targeted modification by glioma initiating cells is enhanced, and the co-loading of siRNA and MIT does not compromise the targeting ability of the exosome drug-loaded system. The exosomes loaded with siRNA and MIT can be successfully taken up by glioma initiating cells. $^D$VAP-Exo/siNotch 1 exhibits a significant silencing effect on the Notch 1 gene. The exosome-targeted drug delivery system demonstrates remarkable antitumor effects *in vivo,* along with good biological safety.

The exosome drug delivery system loaded with mitoxantrone and siRNA can be used to prepare a medicament against brain glioma. Mitoxantrone has an extremely strong killing effect on glioma initiating cells and brain glioma cells. The Notch 1 pathway is highly activated in glioma initiating cells and plays an important role in regulating behaviors, such as proliferation and stemness maintenance, of glioma initiating cells. Targeted tracking of glioma initiating cells and tumor cells is achieved by co-loading a small interfering nucleic acid (siNotch 1) capable of reducing the stemness of the glioma initiating cells and mitoxantrone through exosomes. The sequences of siRNA are set forth in SEQ ID No. 1 and SEQ ID No. 2, and the sequence of the $^D$VAP targeting peptide is set forth in SEQ ID No. 3.

> SEQ ID No.1 UGCCAAAUGCCUGCCAGAATT (sense)
> SEQ ID No.2 UUCUGGCAGGCAUUUGGCATT (Antisense)
> SEQ ID No.3 $^D$S$^D$N$^D$T$^D$R$^D$V$^D$A$^D$P

Hereinafter, the scheme of the present invention is described with reference to the accompanying drawings, wherein the experimental procedures without specific description of the operation steps are performed according to the corresponding product instructions. The instruments, reagents, and consumables used in the examples are commercially available from commercial companies unless otherwise specified.

**Example 1: Construction and Characterization of Exosome-Targeted Drug Delivery System**

Mouse mononuclear macrophages RAW264.7 were provided by Dr. Jingwen Zhao from the Department of Gastroenterology, Tianjin Medical University General Hospital. The cells were cultured using a high-glucose DMEM medium containing 10% exosome-free fetal bovine serum, 1% double antibodies (penicillin-streptomycin), and 1% non-essential amino acids.

1.1 Acquisition and characterization of exosomes

RAW264.7 cells in good growth state were cultured. When the cell density reached 90-95%, the culture supernatant of the mouse mononuclear macrophages was collected. The cell culture supernatant was aliquoted into 50 mL sterile centrifuge tubes, and it was first centrifuged at 4 °C and 2000 g for 20 min to remove live cells and dead cells and then centrifuged at 4 °C and 10000 g for 30 min to remove cell debris. The supernatant was collected. The cell culture supernatant after gradient centrifugation was passed through a 0.22 $\mu$m filter membrane into ultracentrifuge tubes, and it was centrifuged at 4 °C and 100000 g for 70 min. The supernatant was discarded. After it was confirmed that no residual supernatant remained, 150 $\mu$L of PBS was added for resuspension. Every three tubes were combined and resuspended again with 100 $\mu$L of PBS, and finally, the exosomes were collected into 1.5 mL EP tubes. The exosome concentration was detected using a BCA kit, and the exosomes were stored at -80 °C.

The morphology of the exosomes was characterized using a transmission electron microscope. The results are shown in FIG. 2A. The exosomes exhibited a typical "disc-like shape" with a double-layer membrane structure.

5 $\mu$L of the exosomes was diluted with PBS into a solution with a volume of 1 mL, and the particle size distribution of the exosomes was detected by the nanoparticle tracking analysis. The results are shown in FIG. 2B. The particle size of the exosomes was about 124 nm.

The surface-specific molecular markers of the exosomes were determined by the Western blot method. First, the exosomes and cells were lysed with a high-efficiency RIPA tissue/cell lysis solution, placed in a 4 °C refrigerator, and lysed on a shaker for 30 min. The lysate was collected into EP tubes and centrifuged at 12000 rpm for 5 min, the precipitate was discarded, and the supernatant was collected. The protein concentration was determined using a BCA protein assay kit. The extracted cell supernatant and exosome solution were mixed with a 5$\times$ Loading Buffer in a 4:1 ratio, respectively, and the mixtures were heated at 100 °C for 5 min to denature the proteins. The markers CD9, TSG101, and CD63 on the exosome surface were detected by the Western Blot method. The detection results are shown in FIG. 2C, indicating that the exosomes highly expressed CD9, TSG101, and CD63. Through various characterizations of the obtained exosomes, it could be indicated that the exosomes were successfully extracted and isolated.

1.2 Targeted modification and characterization of exosomes

The targeting peptide was modified onto the exosome surface via click chemistry. Firstly, 10 μM DBCO-sulfo-NHS was dissolved in DMSO, and the exosomes resuspended in PBS were added. The mixture was reacted at 25 °C and 120 rpm for 4 h. Subsequently, the reaction mixture was centrifuged in a 50 kDa ultrafiltration tube to remove excess DBCO-sulfo-NHS to obtain DBCO-Exo. Subsequently, the commissioned $^DVAP-N_3$ modified with an azide group was incubated with DBCO-Exo overnight at 4 °C on a shaker at 100 rpm. The next day, centrifugation was performed twice in the ultrafiltration tube with an appropriate amount of PBS to eliminate free peptides, thus obtaining $^DVAP$-Exo.

To verify the successful chemical conjugation of the targeting peptide to the exosomes, the positive rate of TAMRA-$^DVAP$ on the exosomes was detected by flow cytometry to investigate whether the click chemistry reaction was successful. Exosomes modified with TAMRA-$^DVAP$ (Exo-TAMRA-$^DVAP$) were detected, with exosomes not modified with the targeting peptide used as a control, and detection was performed using a flow cytometer. The results are shown in FIG. 3A. About 88.6% of the exosomes displayed a fluorescent signal, indicating successful conjugation of the $^DVAP$ targeting peptide to the exosomes.

1.3 Determination of encapsulation efficiency of drug-loaded exosomes

MIT was dissolved in PBS to prepare a 5 mg/mL stock solution. The stock solution was diluted to obtain MIT solutions with concentrations of 10 μg/mL, 20 μg/mL, 30 μg/mL, 40 μg/mL, 50 μg/mL, 60 μg/mL, 70 μg/mL, 80 μg/mL, and 90 μg/mL. The absorbance of the 9 solutions with different concentrations described above at 627 nm was measured using a microplate reader, and the standard curve for MIT was plotted.

In order to screen for the optimal drug-loading method and conditions, electroporation, pre-co-incubation followed by transfection reagent, and co-incubation combined with transfection reagent methods were compared.

(1) Electroporation method: Exosomes (Exo), MIT, and siRNA were gently mixed in a ratio of 10:8:1. The parameters of the electroporator were adjusted, and electroporation was performed at 1000 V for 20 ms, followed by incubation at 37 °C for 30 min to restore the exosome membrane. Subsequently, an ExoQuick-TC reagent was added, and the mixture was left to stand on ice for 30 min to allow exosome precipitation and then centrifuged at 4 °C and 13000 rpm for 3 min. The supernatant was discarded, and the precipitate was retained. An appropriate amount of PBS was added for resuspension. The obtained system was dialyzed in a dialysis bag for 10 h to obtain exosomes encapsulated with MIT and siRNA (EMR). A 0.5% TritonX-100 solution was added, and the mixture was incubated at room temperature for 15 min to disrupt the exosome membrane structure and release MIT. The encapsulation efficiency of MIT was measured. The absorbance of the solution at 627 nm was measured using a microplate reader, the concentration of MIT was calculated using the standard curve, and the encapsulation efficiency was calculated according to formula 1.

(2) Pre-co-incubation followed by transfection reagent method: Firstly, Exo and MIT were mixed in a ratio of 5:4 and incubated at 37 °C on a shaker at 120 rpm for 30 min, followed by dialysis in PBS at 4 °C for 10 h. Subsequently, siRNA and the transfection reagent Exo-fect were added in a ratio of Exo:siRNA = 10:1. After incubation at 37 °C for 20 min, an ExoQuick-TC reagent was added, and the mixture was left to stand on ice for 30 min to terminate the reaction. The mixture was centrifuged at 4 °C and 13000 rpm for 3 min, the supernatant was discarded, and the precipitate was retained. An appropriate amount of PBS was added for resuspension, and the obtained system was dialyzed in a dialysis bag for 10 h to obtain EMR. A 0.5% TritonX-100 solution was added, and the mixture was incubated at room temperature for 15 min. The encapsulation efficiency of MIT was measured. The absorbance of the solution at 627 nm was measured using a microplate reader, the concentration of MIT was calculated using the standard curve, and the encapsulation efficiency was calculated according to formula 1.

(3) Co-incubation combined with transfection reagent method: MIT, Exo, siRNA, and the transfection reagent Exo-fect were incubated at 37 °C for 20 min in a ratio of Exo:MIT:siRNA = 10:8:1. Subsequently, an ExoQuick-TC reagent was added, and the mixture was left to stand on ice for 30 min. The mixture was then centrifuged at 4 °C and 13000 rpm for 3 min, the supernatant was discarded, and the precipitate was retained. An appropriate amount of PBS was added for resuspension, and the obtained system was dialyzed in a dialysis bag for 10 h to obtain EMR. After dialysis, an appropriate amount of the drug-loaded system was taken, and a 0.5% TritonX-100 solution was added. The mixture was incubated at room temperature for 15 min to disrupt the exosome membrane structure and release MIT. The encapsulation efficiencies of MIT before and after dialysis were measured. The absorbance of the solution at 627 nm was measured using a microplate reader, the concentration of MIT was calculated using the standard curve, and the encapsulation efficiency was calculated according to formula 1.

$$\text{Encapsulation efficiency\%} = \frac{\text{Amount of MIT loaded}}{\text{Amount of MIT used}} \times 100\% (\text{formula 1})$$

In order to calculate the encapsulation efficiency of siRNA in the drug-loaded system, the Cy3 fluorescent dye-labeled siRNA was loaded into the exosomes, and EMR was prepared according to the three methods described above. The corresponding fluorescence values ($E_x$ = 530 nm, $E_m$ = 570 nm) were measured using a microplate reader, and the encapsulation efficiency was calculated according to formula 2.

$$\text{Encapsulation efficiency}\% = \frac{\text{Flupel}}{\text{Flupel} + \text{Flusup}} \times 100\% \text{(formula 2)}$$

wherein $\text{Flu}_{pel}$ and $\text{Flu}_{sup}$ are the fluorescence values of siRNA in the precipitate and supernatant, respectively.

The detection results are shown in Table 1.

Table 1. Encapsulation efficiency of MIT and siRNA in different loading methods

| Loading method | MIT encapsulation efficiency | siRNA encapsulation efficiency |
|---|---|---|
| Electroporation method | 11% | 42% |
| Pre-co-incubation followed by transfection reagent method | 13% | 50% |
| Co-incubation combined with transfection reagent method | 21% | 69.35% |

In the electroporation method, the measured encapsulation efficiencies of MIT and siRNA were 11% and 42%, respectively; in the pre-co-incubation followed by transfection reagent method, the measured encapsulation efficiencies of MIT and siRNA were 13% and 50%, respectively; in the co-incubation combined with transfection reagent method, the encapsulation efficiencies of MIT and siRNA were 21% and 69.35%, respectively. Therefore, the co-incubation combined with transfection reagent method was selected for subsequent loading of MIT and siRNA.

1.4 Preparation and characterization of drug-loaded exosomes

According to the method described in section 1.2 of Example 1, the $^D$VAP peptide was modified onto the exosomes by applying the click chemistry method. Subsequently, according to the co-incubation combined with transfection reagent method described in section 1.3-(3) of Example 1, siRNA and MIT were loaded into the exosomes to obtain $^D$VAP-Exo/MIT/siRNA (VEMR).

The morphology of the obtained VEMR was analyzed using transmission electron microscopy; the particle size of VEMR was analyzed using the nanoparticle tracking analysis (NTA) technology. The results are shown in FIGs. 3B and 3C. The drug-loaded exosomes were round or oval in shape, all possessing a double-layer membrane structure. The particle size of VEMR was around 136 nm. Compared with the untreated exosomes, the morphology of the drug-loaded exosomes showed no significant change, indicating that the exosomes maintained good integrity and their morphology was not damaged during the modification and drug-loading processes. The slight increase in particle size proved that MIT and siRNA were successfully loaded into the exosomes.

1.5 Investigation of drug loading stability

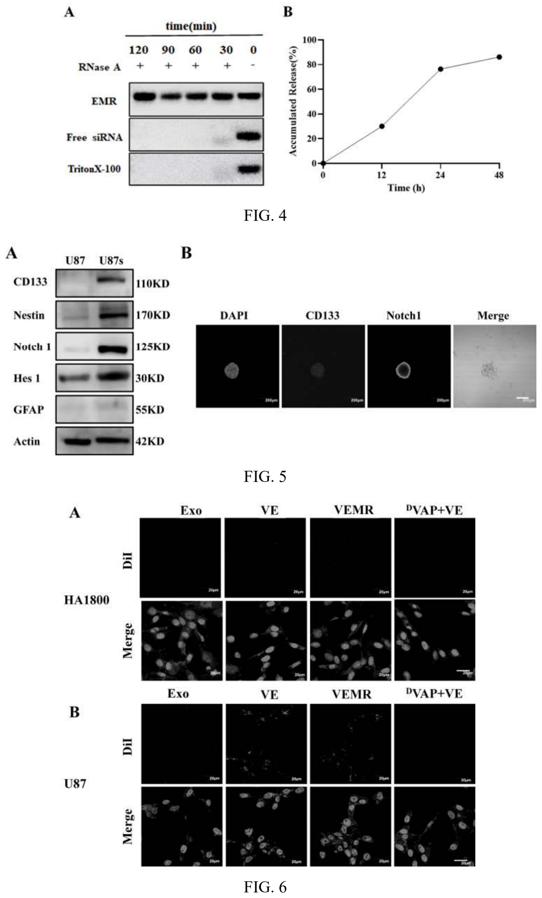

EMR, EMR with membranes disrupted by 0.5% TritonX-100, and siRNA were respectively co-incubated with RNase A at 37 °C for a certain period of time. The degradation of siRNA was then analyzed using agarose gel electrophoresis to investigate the stability of siRNA loaded within the exosomes. Samples incubated with RNase A for 0 h, 0.5 h, 1 h, 1.5 h, and 2 h were thoroughly mixed with a 6× DNA loading buffer, and the mixtures were sequentially added to the loading wells of a 2% agarose gel. Electrophoresis was performed at 120 V for 30 min in the dark, and imaging was performed using a gel imaging system. The results are shown in FIG. 4A, indicating that the free siRNA was mostly degraded at 30 min and completely degraded at 60 min. In contrast, the degradation of siRNA in the EMR with membranes disrupted by using a TritonX-100 solution was similar to that of free siRNA. However, siRNA in the EMR that was not subjected to membrane disruption showed almost no degradation between 0 min and 120 min. This indicates that exosomes provided good protection for siRNA, which thereby exhibited certain stability and was not easily degraded by nucleases.

EMR was prepared, and the sample was placed into a dialysis bag, which was then immersed in 3 mL of a PBS solution for dialysis at 37 °C. The dialysate was sampled at 12 h, 24 h, and 48 h, respectively. The absorbance value of MIT at a wavelength of 627 nm was measured using a microplate reader to investigate the stability of the drug loading. The results are shown in FIG. 4B, demonstrating that MIT was slowly and continuously released within 48 h. This indicates that MIT possessed good stability.

**Example 2: *In Vitro* Biological Function Evaluation of Exosome-Targeted Drug Delivery**

Human normal astrocytes HA1800 were purchased from Shangcheng BeNa Culture Collection, and human glioma cells U87 were purchased from Wuhan Procell Life Science & Technology Co., Ltd. Both of them were cultured using a high-glucose DMEM medium containing 10% fetal bovine serum and 1% double antibodies. Luciferase-labeled mouse glioma cells GL261 were purchased from Shanghai Cell Bank, Chinese Academy of Sciences, and were cultured using a high-glucose DMEM medium containing 10% fetal bovine serum, 1% double antibodies, 1.5 g/L sodium bicarbonate, and 0.11 g/L sodium pyruvate.

2.1 Culture and phenotypic confirmation of glioma initiating cell spheres

U87 cell spheres were isolated using the immunomagnetic bead method. U87 cells in good growth state were first cultured in a DMEM medium. When the cell density reached 80%, the medium was replaced with a DMEM/F12 medium (containing 20 ng/mL EGF, 20 ng/mL bFGF, and B27), and the culture was continued for 3-4 days, with the medium being changed every two days. 0.25% trypsin was added for digestion, and the cells were centrifuged at 4 °C and 1000 rpm for 3 min. The supernatant was discarded. 1 mL of a cell culture medium was added for resuspension and counting, and the number was about $1 \times 10^7$ cells. After centrifugation for 5 min, the supernatant was discarded. The cell suspension was mixed uniformly with 10 $\mu$L of a CD133/PE antibody (1:100), and the mixture was incubated at 4 °C for 10 min, followed by centrifugation. After the unbound primary antibody was removed, 240 $\mu$L of a sorting solution was added to resuspend the cells, followed by the addition of 60 $\mu$L of an FCR blocking agent and 60 $\mu$L of anti-PE magnetic beads. After uniform mixing, the mixture was gently incubated on a shaker at 4 °C for 15 min. Subsequently, 1 mL of a sorting solution was added, and the mixture was centrifuged at 1000 rpm for 10 min. The supernatant was discarded. 500 $\mu$L of a sorting solution was added, and impurities were filtered using a cell strainer. The cell suspension was added to a separation column placed on a magnetic bead separator for sorting. The obtained CD133$^+$ glioma cells were cultured.

Phenotypic confirmation of the glioma initiating cell spheres was performed by the Western blot method. The expression of glioma initiating cell sphere markers (CD133 and Nestin), Notch pathway components (Notch 1, Hes 1), and a cell differentiation marker (GFAP) in the U87 cell spheres was detected. Phenotypic confirmation of the U87 cell spheres was performed using Notch1, Nestin, Notch 1, Hes 1, and GFAP antibodies (1:200). The membrane transfer conditions were 20 V and 20 min.

The Western blot results are shown in FIG. 5A, indicating that the sorted U87 cell spheres highly expressed CD133, Nestin (a stem cell marker), as well as Notch 1 and Hes 1 (Notch pathway components), but the expression level of GFAP (a cell differentiation marker) in the cell spheres was low. The immunofluorescence staining results are shown in FIG. 5B, showing that the U87 cell spheres expressed high levels of CD133 and Notch 1. The above results indicate that the sorted U87 cell spheres conformed to the characteristics of glioma initiating cell spheres and showed almost no differentiation.

2.2 Evaluation of *in vitro* targeting ability

Exosomes were labeled with the membrane dye DiI. Exo and VE were respectively co-incubated with U87 cells, U87 cell spheres, and normal astrocytes HA1800 for 4 h. After the cells were stained with DAPI, the fluorescence intensity within the cells and cell spheres was detected using a confocal laser microscope to investigate the targeting ability of Exo, VE, and VEMR towards U87, U87 cell spheres, and HA1800. The confocal laser microscopy results are shown in FIGs. 6-7. The fluorescence signal in U87 cells and U87 cell spheres treated with $^D$VAP-Exo was stronger than that in U87 cells and U87 cell spheres treated with free Exo, indicating that the uptake of exosomes by U87 cells and U87 cell spheres was enhanced after the targeting peptide modification. The fluorescence signal intensity in the VEMR group showed almost no significant change compared to that in the VE group. This indicates that the loading of MIT and siRNA had little effect on the targeting ability of the exosome drug-loaded system.

A free targeting peptide was used to competitively block the specific binding between the targeting system and the cell surface GRP78 to evaluate the specificity of the targeted delivery by the targeting peptide. The specific method was as follows: U87 cells, U87 cell spheres, and HA1800 cells were first co-incubated with a free targeting peptide for 1 h. After washing with PBS, VE was added, and the fluorescence intensity within the cells was detected using a confocal laser microscope to investigate the effect of receptor blockade on the targeting ability of the system. The results are shown in FIGs. 6-7. After treatment with a free $^D$VAP targeting peptide followed by the addition of VE, no fluorescence signal was observed in U87 cells, U87 cell spheres, and HA1800 cells, indicating that the targeting peptide could specifically bind to the receptor and promote the uptake of exosomes by the cells and cell spheres.

2.3 Uptake of drug-loaded exosomes with targeted modification in cells

U87 cells and U87 cell spheres in the logarithmic growth phase were digested with trypsin, centrifuged, resuspended, seeded into a 24-well plate containing confocal glass slides at $5 \times 10^4$ cells per well, and cultured in an incubator at 37 °C with 5% $CO_2$ for 24 h. PBS, siRNA, MIT, EMR, and VEMR were added to incubate with U87 cells and U87 cell spheres for 4 h, separately. siRNA was labeled with Cy3 fluorescent dye, and MIT itself has a fluorescent effect. The exosome concentration was 10 μg/mL in U87 cells per well, and the exosome concentration was 40 μg/mL in U87 cell spheres per well. After 4 h, the medium in the wells was aspirated, and the cells were washed three times with PBS. Subsequently, the cells were fixed with 4% paraformaldehyde, the nuclei were stained with DAPI, and finally, observations and photography were performed under a confocal laser microscope.

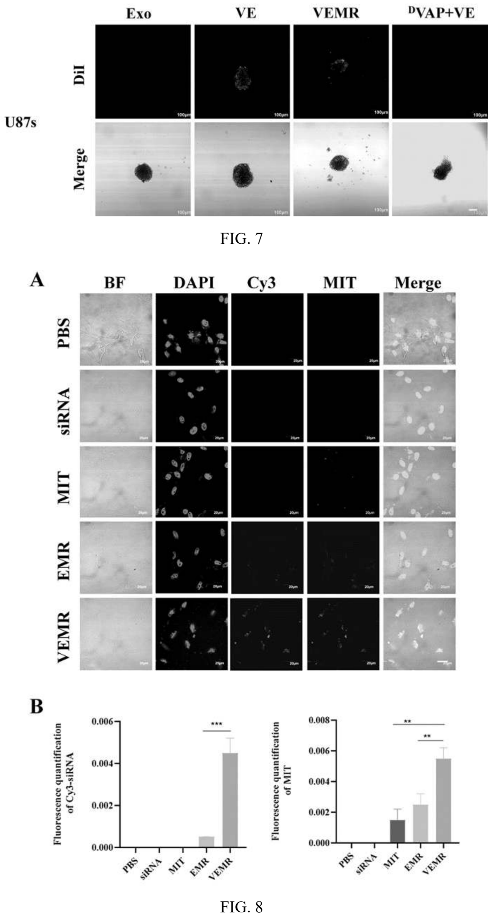

The results are shown in FIG. 8. In U87 cells, almost no red and green fluorescence was detected intracellularly in the free siRNA group and the free MIT group, indicating that free siRNA and free MIT could hardly enter the cells. A small amount of red and green fluorescence signal was observed in the EMR group, while the VEMR group showed strong red and green fluorescence signals, and siRNA and MIT were mainly distributed in the cytoplasm, indicating that the exosomes could successfully carry siRNA and MIT into U87 cells.

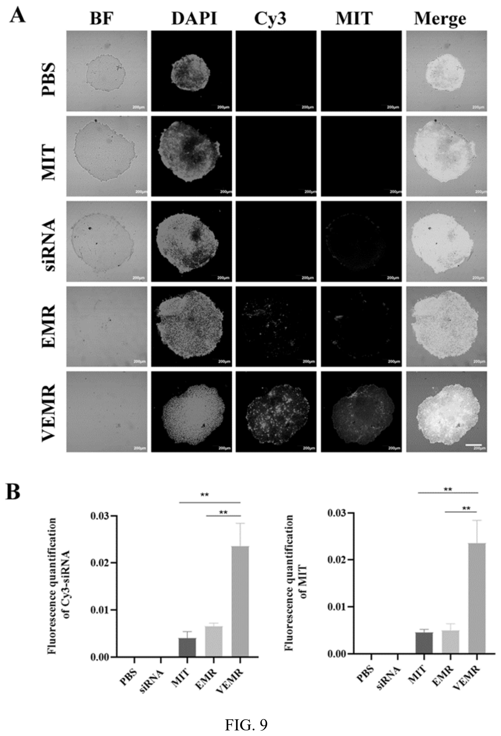

In U87 cell spheres, as shown in FIG. 9, almost no red and green fluorescence was detected inside the cell spheres in the free siRNA group and the free MIT group. A small amount of red and green fluorescence signal was observed in the EMR group, while the VEMR group showed strong red and green fluorescence signals. In summary, these results indicate that the exosomes could successfully carry siRNA and MIT into the cells and cell spheres.

2.4 Uptake mechanism of exosomes with targeted modification in cells

U87 cells and U87 cell spheres in the logarithmic growth phase were digested with trypsin, collected, centrifuged, resuspended, counted, seeded into a 24-well plate containing confocal glass slides at $5 \times 10^4$ cells per well, and cultured in an incubator at 37 °C with 5% $CO_2$ for 24 h. Subsequently, the cells were pretreated for 1 h using different endocytic inhibitors, namely, 50 μM amiloride hydrochloride, 5 mM methyl-β-cyclodextrin (M-β-CD), 10 μg/mL chlorpromazine, and 80 μM dynasore. DiI-labeled $^D$VAP-Exo was added, and the mixture was incubated for 2 h. In order to investigate the effect of temperature on cellular uptake, one group of cells was placed at 4 °C for uptake. After 2 h, the cells were washed three times with PBS, the nuclei were stained with DAPI, and finally, observations and photography were performed under a confocal laser microscope.

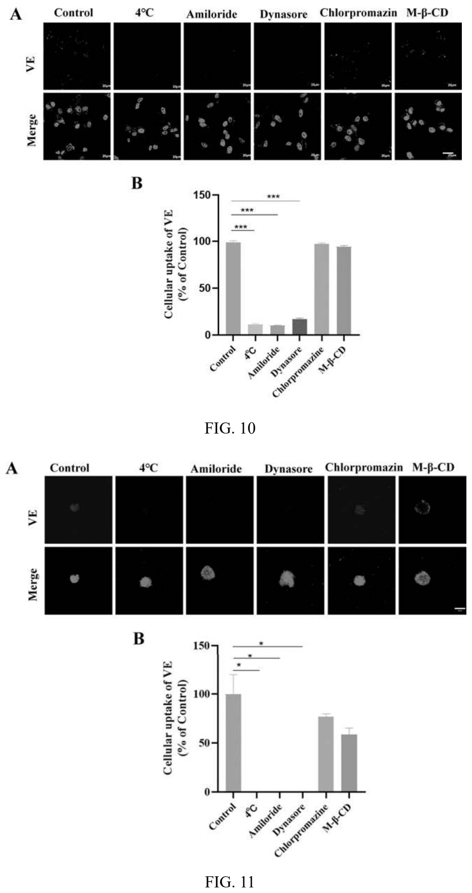

The results are shown in FIG. 10 and FIG. 11. Under the 4 °C condition, the uptake of $^D$VAP-Exo by U87 cells and cell spheres was almost negligible, and the fluorescence signal showed a significant difference compared to that in the control group. This indicates that the cellular entry of $^D$VAP-Exo was an energy-dependent pathway. The caveolin inhibitor M-β-CD and the clathrin inhibitor chlorpromazine did not cause changes in exosome uptake. Amiloride is an inhibitor of $Na^+/H^+$ exchange and macropinocytosis. Dynasore is an inhibitor of GTPase and can inhibit dynamin-mediated endocytosis. In the Amiloride and Dynasore treatment groups, the uptake of VE by cells was reduced, indicating that the cellular entry pathway of the exosomes involved dynamin-mediated endocytosis and macropinocytosis. This suggested that the cellular entry of exosomes was related to energy-dependent processes mediated by dynamin and macropinocytosis.

2.5 Analysis of co-localization of siRNA, MIT, and lysosomes

VEMR was prepared using Cy3-labeled siRNA and MIT for intracellular localization observation of MIT and siRNA. U87 cells and U87 cell spheres in the logarithmic growth phase were digested with trypsin, collected, centrifuged, resuspended, counted, and seeded into confocal dishes to be cultured for 24 h. Subsequently, VEMR was added, and the mixture was incubated for 4 h. The cells were washed three times with PBS and then cultured for another 2 h or 6 h. Subsequently, a Lysotracker (75 nM) solution was added, and the mixture was incubated for 30 min to stain the lysosomes. After the cells were washed three times with PBS, Hoechst was added to stain the nuclei for 30 min. Following three more washes with PBS, the fluorescence distribution was observed under a confocal laser microscope, and the co-localization of MIT, Cy3-siRNA, and Lysotracker fluorescence was analyzed using Image J.

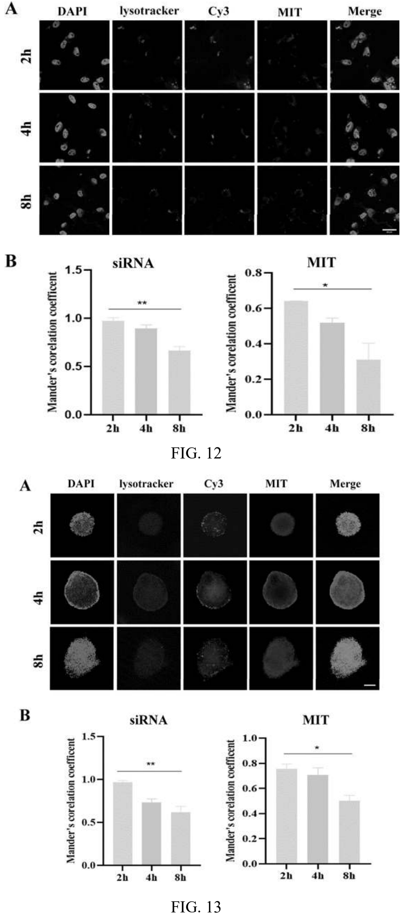

The results are shown in FIG. 12. After the system entered the U87 cells, at 2 h, siRNA and MIT showed substantial overlap with the green fluorescence of the lysosomes. The co-localization coefficient of siRNA with lysosomes was 0.972, and the co-localization coefficient of MIT with lysosomes was 0.64. However, as time progressed, the overlapping fluorescence gradually decreased. At 8 h, the co-localization coefficient of siRNA with lysosomes was 0.67, and the co-localization coefficient of MIT with lysosomes was 0.3. The co-localization coefficients of siRNA and MIT with lysosomes at 2 h and 8 h showed significant differences. In U87 cell spheres (FIG. 13), the co-localization of siRNA and MIT with lysosomes was similar to that of VEMR in U87 cell spheres, indicating that most of siRNA and MIT first entered the lysosomes after being endocytosed by the cells, and then partially escaped from the lysosomes over time.

2.6 Detection of *in vitro* gene silencing effect

Quantitative real-time polymerase chain reaction (qRT-PCR) was used to detect mRNA expression levels. The sequences of siNotch 1 and the negative control (siNC) are shown in Table 2. qRT-PCR was used to detect the silencing effect of siRNA on the target gene. The specific steps were as follows:

(1) Cell plating: U87 cell spheres and GL261 cells were separately seeded into a 24-well plate at $1 \times 10^5$ cells per well. The cells were used for administration when the cell density reached about 80%.

(2) Lipo/siNotch 1 administration: 1 OD of siRNA powder was centrifuged at 3000 rpm for 3 min to pellet the powder at the bottom of the tube, and 250 μL of DEPC water was added for dissolution to formulate a 0.01 nmol/μL stock solution. 3 μL of a Lipofectamine 2000 reagent (Lipo) was first added to 9 μL of a serum-free DMEM medium, and the mixture was left to stand at room temperature for 5 min. 10 μL of the dissolved siRNA solution was added to 90 μL of a serum-free DMEM medium. The diluted siRNA solution was added to the Lipo solution, and the mixture was mixed uniformly by pipetting and left to stand at room temperature for 20 min. The prepared Lipo/siNotch 1 was added to the wells of the plate. After 6 h of culture, the medium was replaced with a DMEM medium containing serum and double antibodies, and the culture was continued for 48 h.

(3) VE/siNotch 1 administration: The preparation was made according to a ratio of VE:siNotch 1:Exo-fect transfection reagent = 10:1:2, and VE/siNotch 1 was added to the cell wells containing a DMEM medium with serum and double antibodies. The mixture was cultured for 48 h.

(4) RNA extraction: An appropriate amount of PBS was added to the wells to wash twice, and 250 μL of Trizol was added to each well.

After standing for 5 min, the mixture was transferred to an EP tube. Chloroform was added, and the mixture was vortexed for 30 s and centrifuged at 12000 g for 15 min. The upper colorless solution was pipetted into a new EP tube. Isopropanol was added, and the mixture was mixed by inverting 3 times, left to stand at room temperature for 10 min, and centrifuged at 12000 g for 10 min. The supernatant was discarded. 75% ethanol was added, and the mixture was vortexed for 10 s and centrifuged at 7500 g for 5 min. The supernatant was discarded. DEPC water was added, and the mixture was incubated in a metal bath at 60 °C for 5 min to dissolve RNA. The RNA concentration was measured using Nanodrop.

(5) Reverse transcription: cDNA was synthesized using a fast reverse transcription kit. A gDNA removal reaction system was formulated according to the instructions, thoroughly mixed uniformly, briefly centrifuged, and incubated at 42 °C for 3 min. A reverse transcription reaction system was formulated according to the instructions (see Table 3 for primer sequences) and added to the gDNA removal reaction solution, and the mixture was thoroughly mixed uniformly. The mixture was heated at 42 °C for 15 min and then at 95 °C for 3 min in a PCR amplifier.

(6) qPCR: The qPCR reaction was performed using a SuperReal fluorescent quantitative premix kit on ice in the dark. 2× SuperReal PreMix Plus, primers, cDNA, and ddH$_2$O were thawed at room temperature and then placed on ice. The reaction system was formulated according to the instructions and reacted in a quantitative real-time PCR instrument (the reaction program was set as follows: 95 °C, 15 min; 95 °C, 3 s; 60 °C, 20 s; 40 cycles). The relative expression level of the Notch 1 gene was calculated according to the $2^{-\triangle\triangle Ct}$ method with β-actin as an internal reference.

Table 2. siRNA sequences

| Name | sense(5'-3') | Antisense (5'-3') |
|---|---|---|
| Notch 1 | SEQ ID No.1:UGCCAAAUGCCUGCCAGAATT | SEQ ID No.2:UUCUGGCAGGCAUUUGGCATT |
| NC | SEQ ID No.4:UUCUCCGAACGUGUCACGUTT | SEQ ID No.5:ACGUGACACGUUCGGAGAATT |

Table 3. Primer sequences

| Name | sense(5'-3') | Antisense (5'-3') |
|---|---|---|
| Notch 1 | SEQ ID No.6:CAGAGGCGTGGCAGACTAT | SEQ ID No.7:GGCACTTGTACTCCGTCAGC |
| NC | SEQ ID | SEQ ID |

(continued)

| Name | sense(5'-3') | Antisense (5'-3') |
|---|---|---|
|  | No.8:TGGCACCCAGCACAAT GAA | No.9:CTAAGTCATAGTCCGCC TAGA |

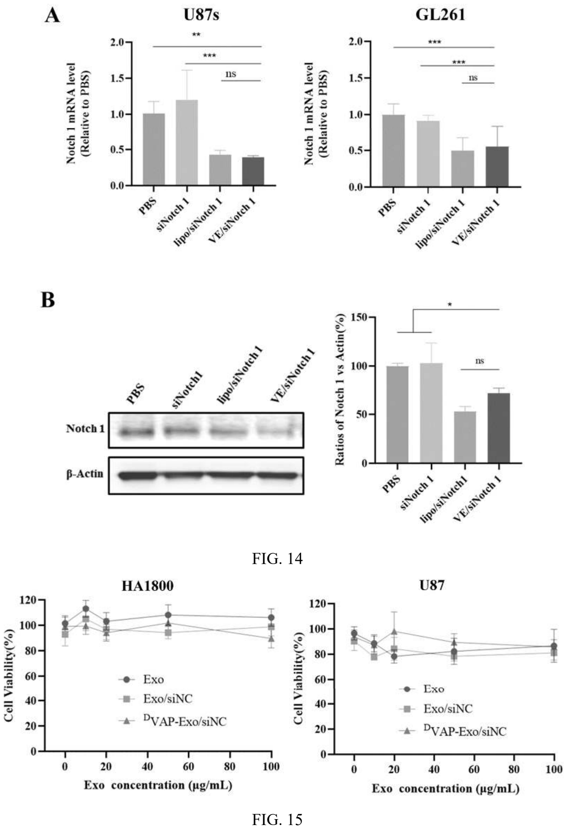

The mRNA expression level was detected using qRT-PCR. The results are shown in FIG. 14A. After VER treatment, the mRNA level of Notch 1 in U87 cell spheres was reduced by 60%, and the mRNA expression level of Notch 1 in GL261 cells was reduced by 45%, showing a significant difference compared to the expression levels of the negative control (free siNotch 1).

U87 cell spheres were seeded into a 12-well plate at $3 \times 10^5$ cells per well. When the cell density reached around 80%, Lipo/siNotch 1 and VE/siNotch 1 were administered. The protein expression level was detected by Western blot. The protein expression level of Notch 1 in U87 cell spheres was detected (FIG. 14B). Consistent with the PCR results, the protein expression level of Notch 1 was significantly down-regulated, and was comparable to the expression level of the positive control group (Lipo/siNotch 1), while the protein expression level of free siNotch 1 showed no significant change. VER could effectively silence the target gene and inhibit the expression of the target protein at the cellular level, providing data support for its subsequent *in vivo* application.

2.7 Evaluation of *in vitro* cell safety

U87 cells and HA1800 cells in the logarithmic growth phase were digested with trypsin, centrifuged, collected, resuspended in a fresh medium, and counted. The cells were formulated into cell suspensions with a concentration of $5 \times 10^4$ cells/mL and seeded into a 96-well plate, with 100 $\mu$L added per well. The plate was then placed in a cell incubator, and the cells were cultured for 24 h. When the cell density reached around 80%, Exo, Exo/siNC, and $^D$VAP-Exo/siNC were added at different concentrations, with the exosome concentrations being 0 $\mu$g/mL, 5 $\mu$g/mL, 10 $\mu$g/mL, 20 $\mu$g/mL, 50 $\mu$g/mL, and 100 $\mu$g/mL, respectively. After another 48 h of incubation, the medium was discarded, and a culture liquid containing a 10% CCK-8 reagent was added. The cells were incubated at 37 °C for another 40 min, and the absorbance values at 450 nm of each well were measured. The wells containing a medium without cells and the drug to be tested were used as the blank group, and the cell group without drug administration was used as the control group. The cell viability rate was calculated according to formula 3:

$$\text{Cell viability rate} \% = (\text{OD}_{\text{experimental group}} - \text{OD}_{\text{blank group}})/(\text{OD}_{\text{control group}} - \text{OD}_{\text{blank group}}) \times 100\% \text{ (formula 3)}$$

The safety of the exosome-targeted loaded siRNA for the normal cell strain HUVEC and the human glioma cell strain U87 was evaluated. As shown in FIG. 15, the commonly used concentration of exosomes in cell experiments was 10 $\mu$g/mL, at which the viability rate of HA1800 cells was around 90%. Furthermore, when the concentration was increased to 100 $\mu$g/mL, the viability rate of HA1800 cells did not change significantly. In U87 cells, as the exosome concentration increased, the viability rate of U87 cells treated with $^D$VAP-Exo/siNC remained no less than 80%, showing no significant difference compared to that in the control group. This indicates that the exosome-targeted drug delivery system possessed good safety.

2.8 Evaluation of *in vitro* cell proliferation

The cell viability rate was detected using the CCK-8 method to investigate the cytotoxicity of formulations of each group. 8 administration groups were set: PBS, MIT, siRNA, MIT+siRNA, VE, VEM, VER, and VEMR. U87 cells and U87 cell spheres were seeded into a 96-well plate at $1 \times 10^4$ cells per well. Each administration group was added to the cells, where the concentration of MIT was 5 $\mu$g/mL, and the concentration of siRNA was 200 nM. The plate was placed in a cell incubator at 37 °C with 5% $CO_2$, and the cells were cultured and incubated for 48 h. The cell viability rate was calculated according to formula 3.

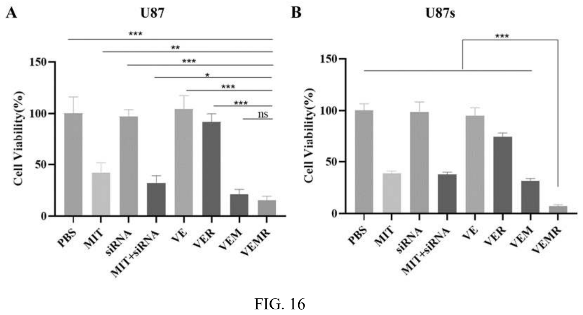

The results are shown in FIG. 16. In U87 cells, due to the low expression of Notch 1 in U87 cells, there was no significant difference in cell viability rate between the VEMR and VEM groups. However, compared to the MIT, siRNA, and MIT+siRNA groups, the VEMR group showed a significant reduction in the cell viability rate. In U87 cell spheres, due to the high expression of Notch 1 in the cell spheres, the cell viability rates of the VER and VEM groups decreased by 30% and 60%, respectively, and the cell viability rate of the VEMR group decreased by about 90%. The above results indicate that VEMR could significantly inhibit the proliferation of U87 cells and U87 cell spheres.

2.9 Evaluation of cell migration ability

A cell scratch assay was used to detect the effect of the exosome-targeted drug delivery system on the migration ability of U87 cells and U87 cell spheres. U87 cells and U87 cell spheres in the logarithmic growth phase were seeded into a 12-well plate at a cell density of $2 \times 10^5$ cells per well. When the cell density reached around 80%, a vertical line was scratched in the center of each well using a 200 μL pipette tip, and floating cells were washed away with PBS. The healing of the scratch was observed under a microscope at 0 h, 12 h, and 24 h after scratching. The scratch distance was quantified using the Image J software, and the scratch distance at each time point was calculated to characterize the inhibitory effect of MIT and siRNA silencing on the migration of U87 cells and U87 cell spheres.

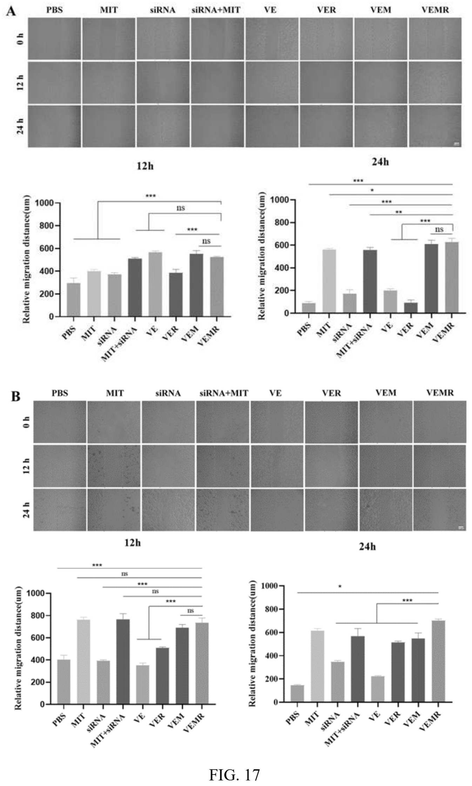

The results are shown in FIG. 17. At 24 h after scratching, in U87 cells, compared to the PBS group and the VE group, the cells in the siRNA group and the VER group had almost completely fused, indicating that siRNA did not affect the normal migration of U87 cells. In contrast, compared to the MIT and MIT+siRNA groups, the "wound" healing in the VEM and VEMR groups was slower, indicating that the VEM and VEMR groups significantly reduced the "wound" healing speed of U87 cells.

In U87 cell spheres, at 24 h after scratching, compared to the PBS group, the cells in the siRNA group had almost completely fused, while the "wound" healing in the MIT and MIT+siRNA groups was slower. Compared to the VE group, the "wound" healing in the VER, VEM, and VEMR groups was slower. Furthermore, compared to VEM and VER, VEMR significantly inhibited "wound" healing. The above results indicate that $^D$VAP could significantly enhance the targeting ability of the system and improve the uptake of siRNA by U87 cells and U87 cell spheres, and MIT and siRNA could jointly enhance the inhibitory effect of the exosome-targeted drug delivery system on the migration of U87 cells and U87 cell spheres.

2.10 Evaluation of cell apoptosis

U87 cells and U87 cell spheres in the logarithmic growth phase were seeded into a 12-well plate at a cell density of $2 \times 10^5$ cells per well, and PBS, MIT, siRNA, MIT+siRNA, VE, VEM, VER, and VEMR were separately added. The mixture was incubated for another 24 h, and floating and adherent cells were collected. The supernatant was discarded by centrifugation, and the cells were washed 3 times with PBS. The cell pellet was resuspended in 100 μL of PBS, stained with Annexin V-APC/DAPI, and then detected using a flow cytometer.

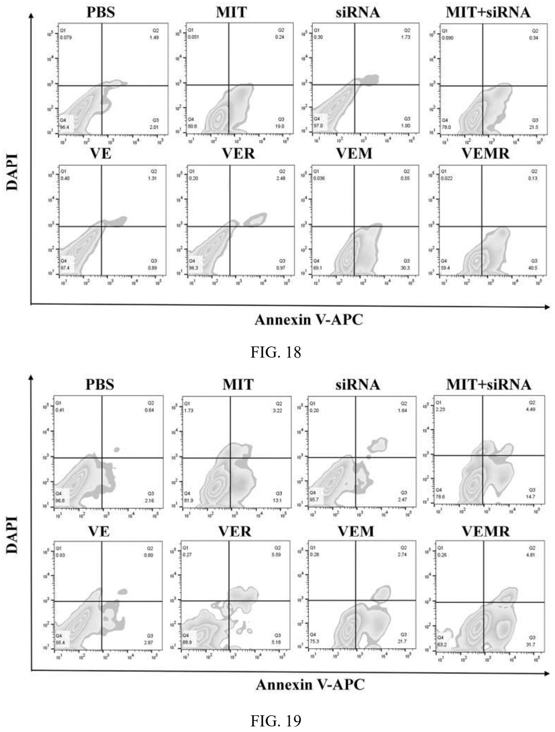

The results are shown in FIG. 18. In U87 cells, compared to the PBS group (3.50%), the cell apoptosis rate in the siRNA group (2.73%) showed no significant change, while the cell apoptosis rates in the MIT and MIT+siRNA groups were significantly increased, at 19.24% and 21.84%, respectively. Compared to the VE group (2.20%), the cell apoptosis rate in the VER group (3.45%) showed no significant change, while the cell apoptosis rates in the VEM and VEMR groups were 30.8% and 40.63%, respectively. Compared to the MIT and MIT+siRNA groups, the cell apoptosis rates in the VEM and VEMR groups were significantly increased.

The results are shown in FIG. 19. In U87 cell spheres, compared to the PBS group (2.80%), the cell apoptosis rate in the siRNA group (4.11%) showed no significant change, while the cell apoptosis rates in the MIT and MIT+siRNA groups were significantly increased, at 16.32% and 19.19%, respectively. Compared to the VE group (3.67%), the cell apoptosis rates in the VER, VEM, and VEMR groups were 10.77%, 30.8%, and 40.63%, respectively. Compared to the MIT and MIT+siRNA groups, the cell apoptosis rates in the VEM and VEMR groups were significantly increased. The above results indicate that $^D$VAP could significantly enhance the targeting ability of the system and improve the uptake of siRNA by U87 cells and U87 cell spheres, and VEMR could significantly promote the apoptosis of GICs and glioma cells.

The experiments described above can prove that the exosome-targeted drug delivery system can exert good gene silencing and antitumor effects at the cellular level, laying a foundation for *in vivo* antitumor applications.

## Example 3: Evaluation of *In Vivo* Antitumor Effect of Exosome-Targeted Drug Delivery System

In this example, an orthotopic brain glioma model was established to investigate the *in vivo* antitumor effect and *in vivo* safety of the exosome-targeted drug delivery system. Balb/c mice (6 weeks old, male, 20±2 g) used were purchased from National Institutes for Food and Drug Control. All animal experiments were performed in accordance with procedures approved by the Animal Ethics and Welfare Committee of Tianjin Medical University, and the procedures were in compliance with the National Institutes of Health (NIH) guidelines for the care and use of laboratory animals.

3.1 Construction of orthotopic brain glioma model

Luc-GL261 cells in good growth state were digested and resuspended in PBS, and the concentration of the cell suspension was adjusted to $1 \times 10^8$ cells/mL. The cell suspension was put onto the ice for later use. Each mouse was inoculated with $1 \times 10^6$ cells. A microsyringe needle was cleaned once with 75% alcohol and then washed twice with PBS.

Surgical instruments were sterilized at high temperature before use. Mice with good vital signs were taken from the animal room and appropriately soothed. The mice were subjected to induction anesthesia by inhalation of a mixed gas of isoflurane and air and then fixed on a brain stereotaxic instrument connected to an anesthesia machine for maintenance anesthesia, and the mouse cranial parts were fully exposed. The mouse scalp was gently incised for 0.5 cm with a scalpel. Along the white cranial suture, a craniotomy was performed 2 mm to the right and 1 mm above the intersection site. The site was gently drilled open with a 5 mL syringe needle, stopping when a puncture sensation was felt, and hemostasis was achieved with a cotton ball. A microsyringe pre-loaded with 10 $\mu$L of GL261 cells was fixed on the brain stereotaxic instrument, directly above the mouse head. The needle was aligned with the drilled cranial opening, and the microsyringe needle was gently inserted slightly into the mouse skull using the Z-axis value of the stereotaxic instrument. The digital display instrument was set to zero. After the Z-axis was moved downward by 4 mm, it was held for 10 s to ensure equilibration within the mouse brain environment. The Z-axis was gently rotated upward by 1 mm to create a space for tumor inoculation. Within 1 min, 5 $\mu$L of the cell suspension was injected into the mouse brain, followed by a 4 min pause to ensure equilibrium within the brain. After the time was up, the microsyringe was gently rotated out. 5 $\mu$L of medical adhesive was drawn, first applied to the cranial wound, and then used to seal the scalp tightly. The mouse was placed on an electric heating pad to recover until awake, and its vital signs were observed the next day.

### 3.2 In vivo imaging experiment

To evaluate the therapeutic effects of different formulation groups in the mouse brain glioma model, on day 12 after tumor bearing, the mice were randomly divided into the following six groups: PBS, MIT, VE, VER, VEM, and VEMR. Administration was performed by intratumoral injection with an administration volume of 10 $\mu$L, where the administration dose of MIT was 3 mg/kg, and the administration dose of siRNA was 0.416 mg/kg. Mice from each group were randomly selected for bioluminescence imaging. On day 19 after tumor bearing, the brain tissue and various organs were dissected from mice in each group for H&E (hematoxylin and eosin) staining and TUNEL staining of the brain tissue and various organs.

The therapeutic effects of different administration groups in the mouse brain glioma model were observed and compared using a small animal *in vivo* imaging system. On days 12, 16, and 19 after tumor bearing, respectively, each mouse was injected with 200 $\mu$L of a luciferase substrate solution and anesthetized, and the fluorescence signal intensity was captured and recorded using an IVIS Spectrum small animal *in vivo* imaging system.

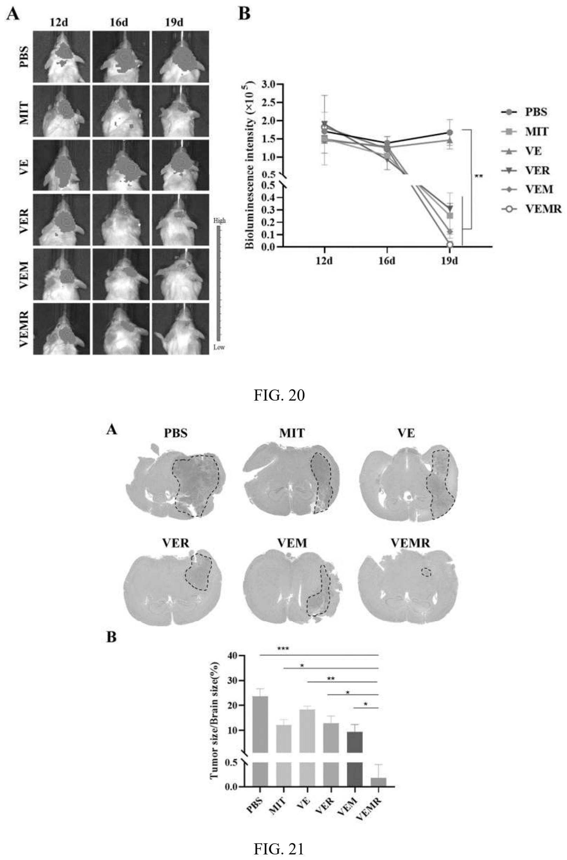

The results are shown in FIG. 20. Mice in each group were administered on day 12 after tumor bearing. The *in vivo* antitumor effect of the exosome-targeted drug delivery system was investigated through bioluminescence imaging (A) and bioluminescence intensity (B) on days 12, 16, and 19 after tumor bearing. On day 12 after tumor bearing, the brain glioma fluorescence values in each group of Balb/c mice all reached above $1 \times 10^5$, indicating the successful establishment of the orthotopic brain glioma model. Within 7 days after administration, the fluorescence values of mice in the PBS group and the VE group gradually increased, indicating rapid brain tumor growth and that the PBS group and the VE group had no significant inhibitory effect on tumor growth. On day 19 after tumor inoculation, the fluorescence values in the VER group and the VEM group were both no more than $0.5 \times 10^5$, showing a significant difference compared to the PBS group, indicating that siNotch 1 and MIT loaded in exosomes could exert a certain gene silencing effect and kill GICs and glioma cells *in vivo.* The VEMR group had the lowest fluorescence value, showing a significant difference compared to the PBS group. This indicates that using $^D$VAP-Exo as a carrier for the combined delivery of siNotch 1 and MIT significantly inhibited tumor growth and possessed a good *in vivo* antitumor effect.

### 3.3 Brain tissue section

On day 7 after administration, mice were randomly selected from each group, and their brain tissues were dissected, fixed in 4% paraformaldehyde for 48 h, and then processed into paraffin sections for H&E staining. Observation and photography were performed using a panoramic digital section scanning microscope to analyze the pathological state of the brain tissues.

The results are shown in FIG. 21. Frozen sections from different formulation groups were collected for H&E staining and observed using a panoramic imaging quantitative analysis system for tissue sections (A). Quantitative analysis of the ratio of tumor area to the entire brain tissue area was performed using the Image J software (B). Both the PBS group and the VE group showed large areas of dense GBM cells, and the ratio of tumor area to the entire brain tissue area was no less than 15% in both groups, indicating rapid tumor growth and no inhibitory effect on tumor growth in these two groups. The tumor area ratios in the VER group and the VEM group were both below 12%, indicating that siNotch 1 and MIT loaded in exosomes could exert a certain gene silencing effect and kill GICs and glioma cells *in vivo.* The tumor area ratio in the VEMR group was below 0.5%, significantly smaller than that in other administration groups, and showed a significant difference compared to that in all other groups, indicating that the combined administration could significantly inhibit mouse brain tumor growth.

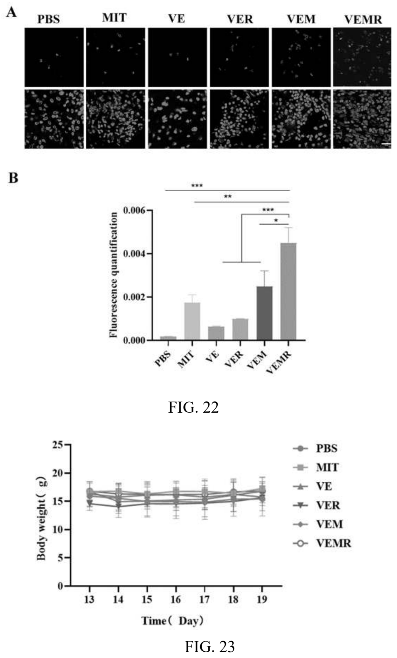

On day 7 after administration, mice were randomly selected from each group, and their brain tissues were dissected and fixed in 4% paraformaldehyde. After fixation was completed, the tissues were placed in a 30% sucrose solution for sugar sinking. When the tissues sank to the bottom, it indicated the completion of sugar sinking. An OCT embedding agent was added into a suitable container, and the container was placed at - 80 °C. After OCT was completely solidified, it was placed in a freezing microtome for sectioning at a thickness of 20 μm. The cut thin sections were spread on glass slides, moistened with an appropriate amount of PBS, and stored at 4 °C. Fluorescence staining to observe cell apoptosis was performed using a TUNEL kit, and imaging was performed using a confocal laser microscope. The results are shown in FIG. 22. Almost no fluorescent signal was observed in the PBS group and the VE group, indicating no induction of tumor cell apoptosis. Compared to the VE group, the green fluorescent signal in the VER group was enhanced, indicating that siNotch 1 could effectively exert its *in vivo* gene silencing effect. The green fluorescent signal in the free MIT group was significantly weaker than that in the VEM group. Compared to the MIT group, VEM had a stronger effect on inducing tumor cell apoptosis. Compared to the VEM and VER groups, the VEMR group showed the strongest green fluorescent signal, with a statistically significant difference, indicating that the combined administration could significantly induce tumor cell apoptosis.

3.4 Investigation of *in vivo* safety of exosome-targeted drug delivery system

The changes in body weight of mice in different administration groups were detected. On day 12 after tumor bearing, body weights of mice in the six groups (PBS, MIT, VE, VER, VEM, and VEMR) were recorded at the same time each day on 1-7 days after administration. The results are shown in FIG. 23. After the successful establishment of the orthotopic brain glioma model, within 7 days after administration, the body weights of mice in each group showed no significant change compared to those in the PBS control group, exhibiting a normal growth trend. This indicates that the exosome-targeted drug delivery system did not affect the normal growth of mice after entering the body.

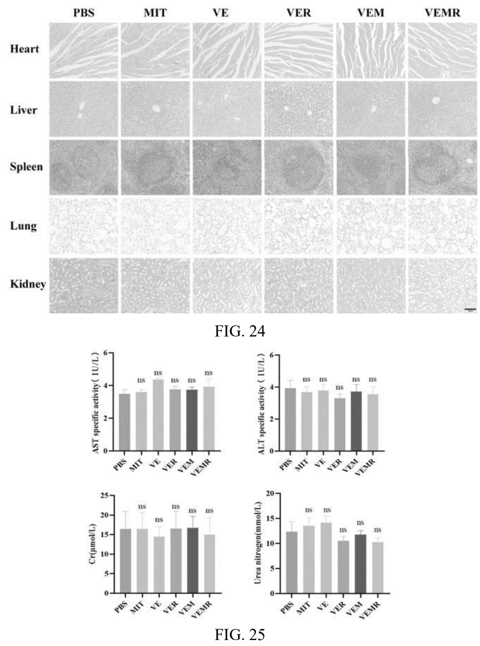

H&E staining was performed on the organs from mice in each group. On day 7 after administration, mice from each group were dissected, and the heart, liver, spleen, lungs, and kidneys were removed, fixed in 4% paraformaldehyde for 48 h, and processed into paraffin sections for H&E staining. The main organs from mice in each group were harvested for H&E staining to evaluate histopathological changes. The results are shown in FIG. 24. The morphological structures of the organs in each group of mice were clear, and no pathological changes such as cell shrinkage, necrosis, or inflammation were observed, similar to the control group. This indicates that the exosome-targeted drug delivery system possessed good *in vivo* biological safety.

The liver and kidney function indicators in mice from each group were detected. On day 7 after administration, blood was collected from the eyeballs of mice in each group, serum was extracted by centrifugation, and liver and kidney function indicators were detected according to an aspartate transaminase (AST) test kit, an alanine transaminase (ALT) test kit, a creatinine (Cr) test kit, and a urea test kit. The detection results of liver and kidney function indicators in mice from each group are shown in FIG. 25. Compared to the PBS group, there were no significant differences in the liver function indicators (AST and ALT) and kidney function indicators (urea nitrogen and creatinine) in any of the groups, indicating that the exosome-targeted drug delivery system did not cause significant adverse effects on the liver and kidney organs of the mice.

The examples of the present invention have been described in detail above, but the content is only the preferred examples of the present invention and cannot be considered to limit the scope of implementation of the present invention. All equivalent changes and modifications made within the scope of the present application shall still fall within the coverage of the patents of the present invention.

**Claims**

1. An siRNA and drug co-delivery system for efficiently targeting glioma initiating cells, wherein a DVAP targeting peptide-modified exosome is loaded with siRNA and mitoxantrone.

2. The siRNA and drug co-delivery system for efficiently targeting glioma initiating cells according to claim 1, wherein sequences of siRNA are set forth in SEQ ID No. 1 and SEQ ID No. 2, and a sequence of the DVAP targeting peptide is set forth in SEQ ID No. 3.

3. A construction method for the siRNA and drug co-delivery system for efficiently targeting glioma initiating cells according to claim 1 or 2, comprising the following steps:

   step 1: modifying an exosome with a DVAP targeting peptide to obtain DVAP-Exo;
   step 2: loading siRNA and mitoxantrone into DVAP-Exo to obtain a drug-loaded exosome with a targeted modification, $^D$VAP-Exo/MIT/siRNA.

4.  The construction method for the siRNA and drug co-delivery system for efficiently targeting glioma initiating cells according to claim 3, wherein in step 1, DBCO is firstly linked to the outer surface of the exosome to obtain DBCO-Exo, and then $^D$VAP-N$_3$ and DBCO-Exo are mixed and incubated to obtain $^D$VAP targeting peptide-modified exosome $^D$VAP-Exo.

5.  The construction method for the siRNA and drug co-delivery system for efficiently targeting glioma initiating cells according to claim 4, wherein 10 μM DBCO-sulfo-NHS is dissolved in DMSO, the exosome resuspended in PBS is added, the mixture is reacted at 25 °C and 120 rpm for 4 h, and excess DBCO-sulfo-NHS is removed to obtain DBCO-Exo;
    $^D$VAP-N$_3$ modified with an azido group is incubated with DBCO-Exo at 4 °C on a shaker at 100 rpm to eliminate free peptides, and $^D$VAP-Exo is obtained.

6.  The construction method for the siRNA and drug co-delivery system for efficiently targeting glioma initiating cells according to claim 3, wherein in step 2, siRNA and MIT are loaded into $^D$VAP-Exo by adopting the co-incubation combined with transfection reagent method.

7.  The construction method for the siRNA and drug co-delivery system for efficiently targeting glioma initiating cells according to claim 6, specifically comprising:

    step 1: uniformly mixing MIT, $^D$VAP-Exo, and siRNA according to a ratio of $^D$VAP-Exo:MIT:siRNA = 10:8:1, adding an Exo-fect transfection reagent, incubating the mixture at 37 °C for 20 min, then adding an ExoQuick-TC reagent, and letting the mixture stand on ice for 30 min;
    step 2: centrifuging the mixture at 4 °C and 13000 rpm for 3 min, discarding the supernatant, retaining the precipitate, adding an appropriate amount of PBS for resuspension, and dialyzing the obtained system in a dialysis bag for 10 h to obtain $^D$VAP-Exo/MIT/siRNA.

8.  Use of the siRNA and drug co-delivery system for efficiently targeting glioma initiating cells according to claim 1 or 2 in preparing a medicament against glioma.

9.  The use according to claim 8, wherein the exosome drug delivery system loaded with mitoxantrone and siRNA is capable of acting on glioma initiating cells and/or glioma cells.

FIG. 1

FIG. 2

FIG. 3

**A**

time(min)

| | 120 | 90 | 60 | 30 | 0 |
|---|---|---|---|---|---|
| RNase A | + | + | + | + | − |

EMR

Free siRNA

TritonX-100

**B**

FIG. 4

**A**

U87    U87s

CD133 — 110KD
Nestin — 170KD
Notch 1 — 125KD
Hes 1 — 30KD
GFAP — 55KD
Actin — 42KD

**B**

DAPI    CD133    Notch1    Merge

FIG. 5

**A**

Exo    VE    VEMR    $^DVAP+VE$

DiI

HA1800

Merge

**B**

Exo    VE    VEMR    $^DVAP+VE$

DiI

U87

Merge

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

## INTERNATIONAL SEARCH REPORT

| International application No. |
| --- |
| **PCT/CN2024/100604** |

| A. | CLASSIFICATION OF SUBJECT MATTER |
| --- | --- |

A61K 47/46(2006.01)i; A61K 47/62(2017.01)i; A61K 47/69(2017.01)i; A61K 31/136(2006.01)i; A61K 31/713(2006.01)i; A61K 9/52(2006.01)i; A61P 35/00(2006.01)i

According to International Patent Classification (IPC) or to both national classification and IPC

| B. | FIELDS SEARCHED |
| --- | --- |

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNABS, CNTXT, DWPI, WOTXT, EPTXT, USTXT, CNKI, 万方, WANFANG, 读秀, DUXIU, PUBMED, ISI_Web of Science, Science Direct, STNext: 天津键凯科技有限公司, 贺慧宁, 杨孟亭, 朱梦玺, 外泌体, 靶向肽, VAP, 米托蒽醌, siRNA, SEQ ID No.1, SEQ ID No.2, SEQ ID No.3, DBCO, 孵育, 透析, 转染, 胶质瘤, exosome, targeting peptide, mitoxantrone, DBCO, incubation, dialysis, transfection, glioma

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | CN 114129734 A (JIANG HAITAO) 04 March 2022 (2022-03-04)<br>claims 1-8 | 1-9 |
| Y | CN 113171468 A (FUDAN UNIVERSITY) 27 July 2021 (2021-07-27)<br>claims 1-3, and description, paragraphs 0043-0044, and table 1 | 1-9 |
| A | CN 109384850 A (FUDAN UNIVERSITY) 26 February 2019 (2019-02-26)<br>claims 1-19 | 1-9 |
| A | CN 111012924 A (AFFILIATED HOSPITAL OF JIANGNAN UNIVERSITY et al.) 17 April 2020 (2020-04-17)<br>claims 1-10 | 1-9 |
| A | CN 112402618 A (NANJING DRUM TOWER HOSPITAL) 26 February 2021 (2021-02-26)<br>claims 1-13 | 1-9 |
| A | CN 113230414 A (QUFU NORMAL UNIVERSITY) 10 August 2021 (2021-08-10)<br>claims 1-10 | 1-9 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **29 September 2024** | **08 October 2024** |

| Name and mailing address of the ISA/CN | Authorized officer |
| --- | --- |
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/100604**

| C. | DOCUMENTS CONSIDERED TO BE RELEVANT | |
|---|---|---|
| Category* | Citation of document, with indication, where appropriate, of the relevant passages | Relevant to claim No. |
| A | CN 117004725 A (TIANJIN CANCER HOSPITAL (TIANJIN MEDICAL UNIVERSITY CANCER INSTITUTE & HOSPITAL)) 07 November 2023 (2023-11-07) claims 1-6 | 1-9 |

Form PCT/ISA/210 (second sheet) (July 2022)

**INTERNATIONAL SEARCH REPORT**

| International application No. |
| --- |
| **PCT/CN2024/100604** |

| Box No. I    Nucleotide and/or amino acid sequence(s) (Continuation of item 1.c of the first sheet) |
| --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2024/100604**

| Patent document cited in search report | | | Publication date (day/month/year) | Patent family member(s) | | | Publication date (day/month/year) |
|---|---|---|---|---|---|---|---|
| CN | 114129734 | A | 04 March 2022 | None | | | |
| CN | 113171468 | A | 27 July 2021 | WO | 2021147917 | A1 | 29 July 2021 |
| CN | 109384850 | A | 26 February 2019 | None | | | |
| CN | 111012924 | A | 17 April 2020 | None | | | |
| CN | 112402618 | A | 26 February 2021 | None | | | |
| CN | 113230414 | A | 10 August 2021 | None | | | |
| CN | 117004725 | A | 07 November 2023 | None | | | |

Form PCT/ISA/210 (patent family annex) (July 2022)